# EUROPEAN PATENT APPLICATION

(11) **EP 2 110 668 A1**
(43) Date of publication of application: **21.10.2009**
(21) Application number: 09005482.6
(22) Date of filing: 17.04.2009
(51) Int. Cl.: G01N 33/543

(54) **Detection test assembly for detecting the presence of a substance in a sample**

(30) Priority: 18.04.2008 US 105590
(71) Applicant: Scheuringer, Kim, A-2500 Baden (AT)
(72) Inventor: Scheuringer, Kim, A-2500 Baden (AT)
(74) Representative: Galloway, Peter David

(57) **Abstract**

A detection test assembly for detecting the presence of a substance preferably a blood substance in a feces sample comprising feces diluted in buffer solution or water is described. The blood substance is detected by acceptors having binding properties for the substance or that may be specifically bound by the substance. Said assembly comprises a reaction field having a mobile first acceptor with visualization properties and a detection field comprising a test field connected to and located downstream from the reaction field having an immobilized second acceptor. After direct application of a prepared feces sample to the assembly, first acceptor forms a complex with blood substance, flows to test field and binds to or is bound by immobilized second acceptor. Accumulation of first acceptor in the test field results in the formation of a visualizable line such as color different from the color of the test field itself. The detection test assembly includes at least two positive internal controls.

## Description

### FIELD OF THE INVENTION

The invention relates to detection test assembly for detecting the presence of a substance in a sample. Specifically, the invention relates to detection test assembly for detecting the presence of a substance in a biological sample. More specifically, the invention relates to a detection test assembly for detecting the presence of a substance in a feces sample, in particular detection of a blood substance. The detection test assembly uses acceptors that have binding properties for the substance or that may be specifically bound by the substance, with detection of the substance shown by formation of a color different from a background color or another visualization technique. The detection assembly also includes at least one positive internal control to ensure reliable and accurate results.

### BACKGROUND OF THE INVENTION

Colorectal cancer is the second leading cause of cancer deaths in Western Europe and in the United States, where this disease claims 56,300 lives per year according to the American Cancer Society.

Fecal occult blood (FOB) tests are used for screening, prevention and for the early detection of colorectal tumors. FOB tests can also indicate a range of gastrointestinal disorders such as hemorrhoids, diverticulitis, Crohn's disease, polyps, fissures, etc. When detected early, colorectal cancer has an extremely high cure rate, which the American Cancer Society estimates being at around 90%.

FOB tests serve as an essential diagnostic tool in the detection of colorectal cancer. As cancers and adenomata bleed intermittently, elevated levels of blood in the stool indicate their presence. Nearly all patients with colorectal cancer exhibit fecal hemoglobin concentrations of > 2mg/g stool while normal subjects have <2 mg/g.

For many years, the basic method for determining blood in stool has been the guaiac method such as Hemoccult, HemoFec or hemoCARE among others (heme-based detection of the peroxidase-like activity of heme).

In the US, Hemoccult is the most commonly used test. However, substances such as red meats, fruits and vegetables high in peroxidase, iron and vitamin C/ascorbic acid can interfere with the test, giving false results. As a result, dietary guidelines must be followed before and during testing. The test period typically lasts 3 days and requires several samplings of stool from the patient. The test must then be mailed or delivered in person to a physician or laboratory for interpretation. As a result, compliance with testing is relatively low.

Guaiac tests are also available for home-use. However, while there is an added convenience of being able to perform the test in the privacy of one's home, the downside is, that all of the interferences for guaiac tests are typically present.

Another method of testing for FOB is by the immunochemical detection of human hemoglobin in the stool. Interferences that are common to guaiac tests are not relevant in such tests. The sensitivity of such tests increases whereas specificities remain high. Such tests are also easier for the patient to perform, as they have no dietary restrictions and require only 1 stool sampling. Additional samplings may be helpful, however, in particular in cases where bleeding is intermittent so that blood does not necessarily appear in a subject's stool every day.

In addition to the foregoing discussion of problems with current methods and tests for detecting blood in fecal matter, other problems with such methods or tests are for instance that they may require multiple-step procedures for processing fecal matter for use in a test, such as centrifugation, filtration, or chemical breakdown with chemicals such as detergents. Current methods or tests may also require the use of two or more test units that need to be mechanically combined prior to or while performing the test, or multiple steps including rinse steps or steps to quench a signal to avoid over-development of a signal and a false-positive result.

Furthermore, other inadequacies of the current methods and tests for detecting blood in fecal matter include problems with monitoring patients who give samples. There is little contact between a physician and patient which can lead to problems with sample collection. Sometimes when a patient collects his own sample, the result is no or less stool which may result in a negative result even if the patient has blood in stool which is a marker for polyps, infections and colon cancer. The current standard built-in control line in such tests consists of an immobilized antibody which detects the mobile labeled antibody used for the test itself. In other words, this control line turns positive when liquid flows trough the test regardless of presence of a sample. However, the test does not detect whether there is fecal matter present in the sample.

Some examples of the above-mentioned patents include U.S. Patent No. 6,531,319 which describes a method of detecting colon cancer wherein fecal matter in a preservative solution is centrifuged or otherwise separated to form a fraction containing glycoproteins. The glycoproteins are precipitated from this fraction, resuspended, and analyzed for a designated cancer marker. U.S. Patent No. 6,221,678 describes a testing device for a substance with a matrix for depositing a sample and a separate testing element for interacting with the matrix. In use, the testing device is for collection of a sample at-home, but the testing element is withheld from at-home use and is used in a laboratory to avoid at-home detection and self-diagnosis of a possible disease state.

U.S. Patent No. 6,221,625 describes an enzyme-labelled immunoassay for detecting a substance including use of a developing solution separate from a sample having the substance and an enzyme-inhibitor solution to prevent excess enzymatic signal formation. U.S. Patent No. 6,221,621 describes a method for screening for cancer by detecting the presence of complement factor proteins or nucleic acids.

U.S. Patent No. 6,057,166 describes a combined filtration and immunoassay system for testing an analyte in fecal matter in part by extracting fecal matter with an extraction reagent detergent and buffer to form a mixture, filtering the mixture, and then detecting the analyte's presence via an immunoassay system. U.S. Patent No. 5,965,375 describes the detection of *Clostridium difficile* using an immobilized anchor molecule and one or more detection molecules both specific to *C. difficile* glutamate dehydrogenase, providing greater clinical sensitivity than a cyclotoxicity assay of *C. difficile* toxin B.

U.S. Patent No. 5,879,951 describes an opposable-element assay device employing unidirectional flow, to avoid for instance clogging of test strips by applying particulate matter. U.S. Patent No. 5,591,645 describes a one step strip test for detecting Group A *Streptococcus* organisms having a visible marker and a strip with overlapping portions that are not in the same plane.

U.S. Patent No. 4,427,769 describes a guaiac immunoassay performed in a cuvette or microtiter plate. U.S. Patent No. 4,200,690 describes an immunoassay without unidirectional flow wherein a desired substance is detected by incubating a membrane having an antibody specific for the substance into a solution of a sample having the substance, rinsing the membrane, then incubating the membrane to show the presence of an antibody-antigen complex. Reissued U.S. Patent No. RE38,088 describes a microtiter plate immunoassay for *H. pylori* in fecal specimens.

### BRIEF SUMMARY OF THE INVENTION

The present invention is directed to a detection test assembly (also referred to as a "DTA" hereafter) for accurately and quickly detecting the presence of a substance, preferably a blood substance, in preferably, a feces sample prepared from fecal matter. The DTA can be used professionally or can be performed as a self-testing device in the privacy of one's home at any time. Such detection can be used for instance to identify clinical marker(s) for diagnosing disease, and/or an abnormal condition of the colon.

A purpose of the detection test assembly of this invention is to detect a fecal matter blood substance having a first and a second portion. Surprisingly, a DTA of the present invention provides a one-step test for determining the presence of a blood substance in an unfiltered feces sample prepared simply by mixing fecal matter with water or a buffer solution. Such a detection test assembly comprises a reaction field comprising a mobile first acceptor having a visualization property, wherein at least one of a first portion of said first acceptor and the first portion of said blood substance has a specific binding affinity for the other, as well as a detection field comprising a test field having a second acceptor immobilized on the test field and wherein at least one of (i) a first portion of said second acceptor and (ii) at least one of (a) the second portion of said blood substance and (b) a portion of a complex formed by the first portion of said first acceptor and the first portion of said blood substance that is different from the first acceptor alone has a specific binding affinity for the other. Furthermore, said first portion of said blood substance is at a location different from said second portion of said blood substance so that said first acceptor, said second acceptor and said blood substance may be specifically bound together simultaneously. Also, said first acceptor does not have a specific binding affinity for said second acceptor and said second acceptor does not have a specific binding affinity for said first acceptor. Also, the test field is connected to and located downstream from the reaction field.

The desired substance is detected if, after direct application of a feces sample prepared as discussed throughout this application to the detection test assembly, the first acceptor forms a complex with the blood substance, flows to the immobilized second acceptor and binds or is bound therein. Accumulation of the first acceptor in the test field results in the formation of a detectable, visualizable signal in the test field such as but not limited to a color different from the color of the test field itself.

The present invention should be competitive with other substance-detection tests and economically feasible. One advantage of the present invention over current substance-detection tests and methods is that fecal matter mixed with a buffer solution or water may be directly applied to a DTA of this invention. The present invention does not require further processing of the feces sample, such as filtering, centrifuging, chemically breaking down feces with a detergent, or other processing required for instance by state-of-the-art tests. A DTA of this invention requires only one unit, in contrast to separate units that must be joined together for the DTA to provide a complete result. Further, the DTA of this invention does not necessarily require the overlap of sections of a test strip in order to permit flow. Also, the present invention does not include rinsing, quenching, guaiac dying, eluting, or other steps besides directly applying a properly prepared feces sample to the DTA and reading the result. The result of a DTA may be read visually or optionally by using a meter.

Another goal of the invention is the reliable and safe operation of such a detection test assembly, preferably in a test kit. The present invention is also directed to a kit comprising a container and a DTA of this invention as well as one or more of an instrument for transferring fecal matter, a second container, a housing and a buffer solution, as discussed throughout this application. Preferably, the second container has a means for measuring the buffer solution. More preferably, a volume of the buffer solution is pre-measured into the container. Even more preferably, the container may be closed and re-opened for instance with a screw-cap or other cap so that buffer solution and fecal matter therein may be mixed together by shaking the container.

Another goal of the invention is to provide at least one positive internal control to provide accurate and reliable results. For example, the positive control detects something that is common to fecal matter. Thus, the positive control detects whether there is fecal matter in the sample. The invention can also have a second positive control that detects something, other than a component of fecal matter, in the feces sample substance. In other words, this control line turns positive when liquid flows trough the test regardless of presence of feces.

The present invention is also directed to a method for detecting a substance, preferably a blood substance, in fecal matter comprising the steps of preparing a feces sample and applying the feces sample to the DTA.

Other features of this invention will be in part apparent and in part pointed out hereafter.

### BRIEF DESCRIPTION OF THE DRAWINGS

Figure 1 is a side view illustration of a detection test assembly of the present invention.
Figure 2 is a side view illustration of a detection test assembly of the present invention.

Figure 3 is a photograph of a top view of a detection test assembly of the present invention after application of a prepared feces sample showing a positive result for a blood substance of a feces sample at the test field and a control result at the control field.

### DETAILED DESCRIPTION OF THE INVENTION

The present invention is directed to a detection test assembly for detecting the presence of a substance, preferably, in a feces sample comprising fecal matter diluted in a buffer solution or water. The substance is preferably a blood substance and is detected by using acceptors having binding properties for the substance or that may be specifically bound by the substance. Said assembly comprises a reaction field having a mobile first acceptor with visualization properties and a detection field comprising a test field connected to and downstream from the reaction field having an immobilized second acceptor. The desired substance is detected if, after direct application of a prepared feces sample to the detection test assembly, the first acceptor forms a complex with the substance, flows to the test field and binds to or is bound by immobilized second acceptor therein. Accumulation of the first acceptor with visualization properties in the test field results in the formation of a visualizable line such as color different from the color of the test field itself.

The following definitions and discussion refer to the detection test assembly of this invention unless the context demands otherwise. If not expressly defined, a term of this invention should be defined as implicitly described throughout this application.

A "detection test assembly", or "DTA", of the present invention is a device for detecting a substance, preferably a blood substance, in a biological sample, the biological sample is preferably prepared by diluting fecal matter in a buffer solution or water. Each detection test assembly comprises a reaction field and a detection field comprising a test field. The reaction field has a mobile first acceptor that has a specific binding affinity for, or will be specifically bound by, at least a portion of the blood substance the DTA is designed to detect. In other words, at least one of a first portion of said first acceptor and a first portion of said blood substance has a specific binding affinity for the other. A "portion" in this and similar contexts refers to an area, usually a surface area, such as an epitope. "Specific binding affinity" as used in this application refers to the customary meaning of the term in the art, that is, binding with greater affinity than binding with other chemicals overall. For instance, if the first acceptor has a specific binding affinity for a first portion of the blood substance, the first acceptor will bind to said first portion with greater affinity than to other portions of the substance and than other substances in general. Preferably, specific binding affinity refers to a very high affinity such as seen with antibodies and antigens, sometimes referred to in the literature as essentially "irreversible".

The test field has an immobilized second acceptor that has a specific binding affinity for, or will be specifically bound by, at least a portion of the blood substance the DTA is designed to detect, or a portion of the first acceptor-substance complex that is different from the first acceptor alone. In other words, at least one of a first portion of said second acceptor and a second portion of said blood substance has a specific binding affinity for the other. Or, at least one of a first portion of said second acceptor and a portion of the first acceptor-substance complex that is different from the first acceptor alone has a specific binding affinity for the other. The first portion of the blood substance is at a location on the blood substance that is different from the second portion of the blood substance so that said first acceptor, said second acceptor and said blood substance may be specifically bound together simultaneously (at the same time once the first acceptor-substance complex has moved to the test field). For instance, the first acceptor and second acceptor cannot be the same monoclonal antibody, as the first and second acceptors would then have to compete for the same epitope on the substance to be detected, and as the first acceptor may saturate all of these shared binding sites and therefore could not accumulate in the test field. Similarly, the first acceptor and second acceptor cannot be the same acceptor if a similar result would occur.

In a DTA of this invention, a reaction field and a detection field comprising a test field are connected so that at least part of a prepared biological sample, once applied to a DTA, flows from or through the reaction field and into the test field. Preferably, the detection test assembly also has a sample application field upstream from the reaction field where the sample may be applied. The prepared biological sample may be applied to the reaction field directly, or to another field upstream from the reaction field. Preferably, the biological sample is applied to a sample application field, and then flows through the reaction field to the test field.

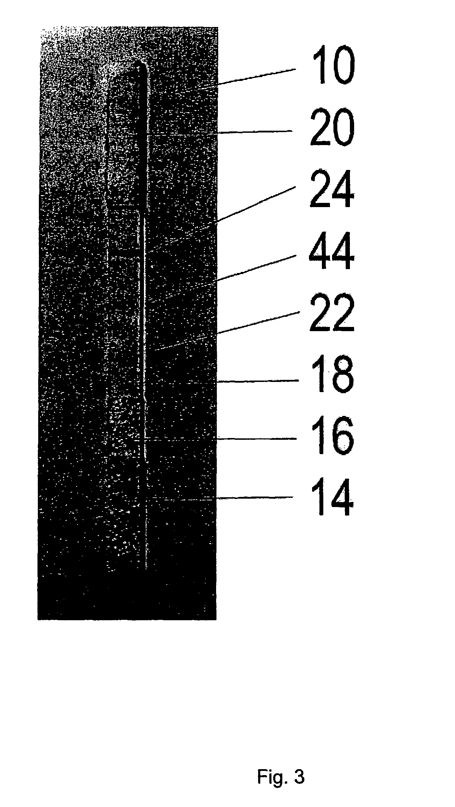

A detection test assembly may be shaped in a form of a rectangular strip such as the strip seen in Figure 3A. Such a strip is economically preferable for the simple manufacture of sheets and subsequent cutting of a sheet into said strips. Other shapes including but not limited to a circular shape or irregular shape are also suitable for a DTA of the present invention.

Preferably, a DTA has a rectangular shape that is 1 cm to 20 cm long and 0.1 cm to 5 cm wide, more preferably 4 cm to 10 cm long and 0.2 cm to 1 cm wide, and most preferably 6 cm long by 0.3 cm wide or 9 cm long by 0.5 cm wide. A DTA may be prepared in a much wider sheet, for instance about 30-50 cm wide or long, and cut to a preferred size. The thickness of a DTA may vary based in part for instance on the material used for a given field.

A DTA may be made of one piece of material, or of several pieces of material connected together so the feces sample may flow through from the reaction field to the test field. Preferably, several pieces of material are connected together by a supporting backing. When ready for use, the DTA is a single unit. A "single unit" in this sense includes all of the essential elements together so that a DTA may be used as a one-step test wherein the step is to apply prepared feces sample to a DTA and a result is shown thereafter. The DTA is a one-step test because the only step in performing the test is to apply a feces sample (fecal matter diluted in buffer or water) to the DTA. Use of the DTA could also be considered a two-step test, by counting the step of preparing the feces sample as a first step and applying the feces sample to the DTA as a second step. However, upon applying the prepared biological sample to the DTA, no further step is needed to achieve a readable result.

A "substance" to be detected by a DTA from a biological sample includes but is not limited to whole or breakdown products of a blood substance, bacteria (i.e. *H. pylori*), hormones, tumor markers such as for instance m2pk or haptoglobin, antibodies, drugs, food, other chemicals (hormones, tumor markers, antibodies, drugs, food and other chemicals may also be considered a blood substance). A DTA that detects these substances may be used for instance to test for pregnancy, ovulation, drug use, drug clearance, liver malfunction, intestinal flora, and/or other conditions related to a subject's physical status.

In addition to the above discussion, a "blood substance" includes but is not limited to a blood component, or a modified blood component, or a degraded blood component or any combination thereof. A blood component may include but is not limited to lipoprotein, hemoglobin, erythrocyte, electrolyte, immunoglobulin, albumin, and other components typically expected to be found in blood. A blood component may include but is not limited to a hormone, drug, antibody, truncated protein, tumor marker, or other chemical that may be, but is not necessarily, usually present in blood. A modified blood component includes but is not limited to a blood component that has been modified in some manner, physiologically or otherwise, including but not limited to a modification such as glycosylation. One example of a modified blood component is glycosylated hemoglobin. A degraded blood component includes but is not limited to a blood component that has been degraded in some manner, natural or otherwise, including but not limited to hemoglobin degradation products such as hemoglobin alpha chain, hemoglobin beta chain, and the heme system. Preferably, a blood substance to be detected in a fecal sample is a clinical marker for cancer, more preferably colon cancer. Such clinical marker may be, without limitation, hemoglobin, m2pk, haptoglobin.

"Feces" or "stool" or "fecal matter" is any material discharged through the anus of a human or animal. Feces useful in this invention may be a hard or soft solid or in liquid form. Preferably, the fecal matter used to prepare a feces sample is a hard (formed) or soft (loose) solid, more preferably a hard (formed) solid.

A "feces sample" or "prepared feces sample" is fecal matter diluted in a buffer solution or in water, preferably a buffer solution. A feces sample may be applied to a DTA of this invention. Once applied to a DTA, a feces sample is able to flow across a DTA reaction field and into a test field in order for a substance, preferably a blood substance, to be detected by the DTA. A feces sample is applied to a DTA in order to detect a substance such as a blood substance.

In preparing a feces sample, fecal matter is added to water or a buffer solution, preferably a buffer solution, and stirred or shaken in order to help break apart and/or dissolve and/or dilute the fecal matter. Preferably, fecal matter will be collected from a toilet or other collection device by using an instrument such as a long toothpick or tongs or spoon or other device to take a small portion of fecal matter and transfer it into a container such as a vial holding buffer solution or water, preferably buffer solution. Even more preferably, the fecal matter is collected immediately after a bowel movement. A device such as but not limited to the instrument used to collect feces may be used to stir and/or mechanically break up feces to prepare a feces sample. Preferably, the amount of fecal matter to be used to prepare a feces sample is 0.1 mg to 10 g, more preferably 1 mg to 1 g, and even more preferably 10 mg to 100 mg. Preferably, the volume of buffer solution or water used to prepare a feces sample may be adjusted based on the amount, density and/or thickness or viscosity of the feces collected, for instance so that the feces sample is not too thick to flow through the DTA. More preferably, the fecal matter is diluted in 0.2 to 20 ml of buffer solution, even more preferably in 1 to 10 ml of buffer solution, even more preferably in 2 to 4 ml of buffer solution, and most preferably in 2.5 ml of buffer solution. Larger buffer volumes may be difficult to handle and may need more fecal matter than smaller buffer volumes. Preferably, the fecal matter is diluted in a ratio of fecal matter to dilution volume of, based on calculation of mg fecal matter / ml buffer solution or ml water, 0.1 mg/ml to 100 mg/ml, more preferably 1 mg/ml to 10 mg/ml, more preferably 2 mg/ml to 6 mg/ml, more preferably 3 mg/ml to 5 mg/ml, and still more preferably 4 to 4.6 mg/ml.

The pH of a buffer solution for preparing a feces sample is preferably pH 4 to 10, more preferably pH 6 to 8, more preferably pH 7 to 7.8, and most preferably pH 7.4. The salt concentration of the buffer solution may vary according to the substance a DTA is to detect. Preferably, the total salt by weight in 1 L of water is 0 to 100 g salt, even more preferably, 3 to 12 g salt, and even more preferably, 7 to 11 g salt. The inclusion of 7 to 11 g salt in 1 L of water is considered a physiological range of salt for the purposes of this invention. For a DTA for detecting a cytosolic blood substance such as a cytosolic protein, the buffer solution preferably has 10 to 14 g salt and even more preferably 11 to 12 g salt. A physiological salt concentration of about 7 to 11 g total salt in 1 L of water is preferable when detecting a blood substance such as a hormone. When detecting a cytosolic protein such as hemoglobin, while not being bound by theory, a higher or lower salt concentration is preferred as such higher concentration may help lyse the red blood cells in the blood and release hemoglobin from the red blood cell cytosol. Very high salt concentrations may interfere with specific binding of immunoassay components. The buffer solution may also include for instance bovine serum albumin ("BSA"), or other proteins or substances including but not limited to Tween 20 that for instance may reduce non-specific acceptor binding by saturating non-specific binding sites on DTA surfaces that may non-specifically bind any protein including antibodies. The buffer solution may also include for instance salts and/or non-salts such as preservatives, anti-microbial agents, anti-autofluorescence agents, and/or other chemicals, including but not limited to sodium azide (NaN3), ethylenediamine tetraacetic acid (EDTA), benzalkonium chloride, chlorhexidine, thimerosal and sorbic acid, for instance to help avoid interference with a DTA test field result.

A "field" is part of the physical hardware and flow system of the DTA. All DTA fields are physically connected to each other. A DTA field may be connected to another field, directly or indirectly, so that at least part of a feces sample applied to a DTA may flow from one field to the next. For instance, a reaction field and detection field may be indirectly connected for instance by placement of another field between them. Preferably, one DTA field is directly connected to another field by abutting, more preferably seamlessly abutting, another field, so that two or more DTA fields are in essentially the same plane and are in direct contact with each other. The fields may overlap, but do not require overlap. Where overlap occurs, preferably such overlap is 0.5mm to 3mm, more preferably 1 mm. Preferably, fields are glued onto a supporting backing, fixing their relation to one another while retaining flow from one field to the next. In a DTA of this invention, feces sample must be able to flow from reaction field to test field. Flow in the direction of reaction field to test field is called "downstream" flow. A test field is therefore also considered to be located "downstream" of a reaction field in a given DTA; a reaction field is located "upstream" of a test field.

Preferably, a DTA field directly abuts (borders on and directly contacts) another field, and more preferably seamlessly abuts (abuts by directly contacting along an entire edge, with or without overlap) in the proper flow order as described throughout this application. Even more preferably, the entire DTA has a substantially uniform length and width, so that one field seamlessly abuts another field. See for instance Figure 3A wherein a DTA is pictured as having a substantially uniform length and width, and wherein one field seamlessly and directly abuts one or more other fields. Another means for connecting fields of the DTA is by inserting an "empty" field or other structure having no substantive function with regard to detecting the substance to be detected by a DTA but that allows a prepared feces sample to flow through to another field, preferably to a field having a detection function.

A field may be made of one or more substances, preferably only one substance, including but not limited to fiberglass, nitrocellulose, paper, polycarbonate, polysulfone, nylon. A field is sized proportionately to the size of the DTA in which it occurs; the total size of a given DTA must comprise the sizes of all of the fields of that DTA.

The term "sample application field" means, but is not limited to, a field for applying a feces sample to a DTA. If present, this field is located upstream of a reaction field of a DTA. Preferably, a sample application field is made of fibreglass.

The term "reaction field" means, but is not limited to, a field having a mobile first acceptor. A feces sample may be applied directly to a reaction field. The feces sample must be able to flow from reaction field to test field. Preferably, a reaction field is made of fibreglass.

The term "test field" means, but is not limited to, a field having immobilized second acceptor. When the blood substance to be detected is present in detectable amounts in the feces sample, the test field will show a positive result for instance in the form of a color different from the color of the test field or through some other visualization technique. Preferably, the color of the test field is a light color such as white or light yellow or light pink or light blue or light red or light tan, most preferably white. Preferably, the color resulting from a positive result is a dark color. Preferably, the second acceptor is immobilized in the test field in the shape of a straight line, so that when first acceptors with visualization properties bind to second acceptors, a positive result will appear in the test field for instance as a colored line. When the substance to be detected by the DTA is not present, the test field will not show a result in the form of for instance a line of color different from the color of the test field. Preferably, a test field is made of nitrocellulose membrane.

The term "first control field" means, but is not limited to, a field having immobilized third acceptor. A first control field is preferably included in a DTA at least because a first control field will confirm that fecal matter is present in a feces sample. A first control field will also show the DTA is working properly by showing for instance a line of color different from the color of the test field when a feces sample is applied to the DTA and that the feces sample containing the fecal matter has flowed through the DTA to the first control field. However, the color may be the same as the color indicated at the test field. If a feces sample applied to a DTA does not contain the blood substance the DTA is meant to detect, then no positive result will appear in the DTA test field. However, where a first control field is present, a first control result will appear if fecal matter in the feces sample is present. For instance, a color different from the color of the test field will appear in the first control field, regardless of and independent from the presence of the substance to be detected. A result in the first control field assures a DTA user that fecal matter is present in the feces sample. Preferably the first control field is downstream of the test field; however, the first control field may be positioned anywhere on the detection field. Preferably, third acceptor is immobilized on the first control field in the shape of a line, so that a different color appears in the form of a line (" first control line"). Preferably, a first control field is made of nitrocellulose membrane. Preferably, the first control field is made of the same nitrocellulose membrane as the test field.

The term "second control field" means, but is not limited to, a field having immobilized fourth acceptor. A second control field is preferably included in a DTA at least because a second control field will show that a DTA is working properly by showing for instance a line of color different from the color of the test field when a feces sample is applied to the DTA and the sample has flowed through the DTA to the second control field. If a feces sample applied to a DTA does not contain the blood substance the DTA is meant to detect, then no positive result will appear in the DTA test field. However, where a second control field is present, a second control result will appear. For instance, a color different from the color of the test field will appear in the second control field, regardless of and independent from the presence of the substance to be detected. A second control field assures a DTA user that the DTA is working correctly. Preferably the second control field is downstream of the first control field; however, the second control field may be positioned anywhere on the detection field. Preferably, fourth acceptor is immobilized on the second control field in the shape of a line, so that a different color appears in the form of a line ("second control line"). Preferably, a second control field is made of same nitrocellulose membrane as the first control field. Preferably, the second control field is made of the same nitrocellulose membrane as the test field and the first control field.

The term "detection field" means, but is not limited to, a field comprising the test field. A detection field comprises a test field and may consist essentially of or consist of a test field. A detection field is where a test result is read, so that a positive test result is visible, or may be read by, a human eye or electronic reading device. If a first or second control field are present, the first or second control fields are also in the detection field, preferably downstream from the test field. A detection field will not include any field type other than a test field and at least the first and second control fields, however, a detection field will preferably include some extra flow space as desired on either side of any field and more preferably between test field and the first control field and between the first control field and second control field. Preferably, a detection field is made of nitrocellulose membrane.

The term "soaking field" means, but is not limited to, a field occurring downstream of a test field. When in use, a soaking field may help facilitate and maintain feces sample flow through a DTA by absorbing an excess of feces sample. Preferably, a soaking field provides a sink or other area for flow to end beyond the test field. Preferably, a soaking field is made of porous material able to absorb a liquid such as paper, or of an absorbent matrix such as a silicagel matrix.

The term "first acceptor" includes but is not limited to an antibody, antigen, hapten, immunoglobulin, T-cell, or any chemical or biological substance having a specific binding affinity for, or that will be specifically bound by, a substance, preferably a blood substance, to be detected by a DTA. As discussed above, once bound, specific binding is preferably not easily reversible, and is preferably considered to be irreversible according to the customary meaning of the term. A first acceptor is mobile and is localized in the reaction field of an unused DTA so that, when a feces sample is applied to and flows through a DTA in a downstream direction, first acceptor may flow with the feces sample downstream.

A first acceptor must have a "visualization property" so that, when first acceptors accumulate near each other for instance in a test field, such accumulation causes a detectable physical property different from background or other detectable property already existing in a test field or a control field, for instance by causing formation of color, change of color, or even in some cases a decrease of color when detection occurs therein. This different physical property is detectable by a human eye or electronic device. As discussed throughout this application, a visualization property is not necessarily restricted to only first acceptor. However, preferably first acceptor has a visualization property. A visualization property may be inherent to for instance first acceptor, such as for instance when first acceptor is an autofluorescent compound, or may be from a label chemically attached to first acceptor. Preferably, this property is from a label chemically attached to first acceptor such as colloidal gold or colored latex particles using standard protocols. Such labels may be conjugated to first acceptor (or control acceptor where applicable) using standard protocols. When substance to be detected is present, first acceptor and substance specifically bind to form first acceptor-substance complex. Such complex flows to the test field. Accumulation of first acceptor-substance complex in the test field (by binding to second acceptor as discussed throughout this application) results in a positive test result that is visible for instance as a color to a human eye or electronic reading device. Another label having a visualization property that may be attached to/conjugated to a first acceptor (if the visualization property is not inherent) is a magnetic bead or other magnetic particle. Accumulation of first acceptor with a magnetic bead could be visualized, or read by, an electronic device that detects a magnetic field or conductivity. Another label having a visualization property that may be attached/conjugated to a first acceptor is an ordinary chemical color dye such as TMB. Labels having visualization properties are not restricted to being conjugated only to first acceptors, but are useful as discussed throughout this application.

Preferably, except as expressly stated otherwise in this application, an acceptor of this invention is an antibody. An antibody for use in a DTA of this invention may be monoclonal or polyclonal or any combination thereof, and may be obtained from an animal, chemical or other source. More preferably, an antibody used in a DTA as a first acceptor or a second acceptor is a monoclonal antibody, with each antibody specific for a different epitope of the blood substance to be detected by the DTA. If present, preferably an antibody used as a third acceptor is a polyclonal antibody, to provide for a broader detection of control substances. Preferably, the antibody concentration for soaking or coating a field of a DTA of this invention is about 0.1 mg antibody/ml to 25 mg/ml, for instance, about 0.1 mg/ml, about 0.25 mg/ml, about 0.5 mg/ml, about 1 mg/ml, about 2.5 mg/ml, about 5 mg/ml, about 10 mg/ml, about 15mg/ml, about 20mg/ml, about 25 mg/ml, or any concentration or concentration range therein. For other acceptors of this invention, one of skill in the art will easily be able to determine acceptable concentrations based on the strength of the specific binding affinity of the acceptor for substance or substance for acceptor. Other acceptors may be, for instance, biotin, avidin, streptavidin, magnet or magnetic substance, iron. Such substances have a specific binding affinity for, for instance and respectively, avidin, biotin, biotin, heme, magnet or magnetic substance.

The term "second acceptor" includes but is not limited to an antibody, antigen, hapten, immunoglobulin, T-cell, or any chemical or biological substance having a specific binding affinity for, or that will be specifically bound by, a substance, preferably a blood substance, to be detected by a DTA, or having a specific binding affinity for, or that will be specifically bound by, part of a first acceptor-substance complex formed, for instance, in the reaction field or otherwise in the DTA within or between the reaction field and the test field. A second acceptor has no specific binding affinity for first acceptor and a first acceptor has no specific binding affinity for second acceptor. This is because specific binding between first and second acceptor, without the presence of a blood substance, would simply mimic a possible control of this invention. A second acceptor is immobilized, preferably in the shape of a line, in the test field of a DTA.

A second acceptor does not have the visualization property of first acceptor, and is not for instance labelled with colloidal gold, as the second acceptor typically forms the line or other shape but not the color of a positive test result. In an alternative embodiment, a second acceptor may have a visualization property so long as, when first acceptor binds to second acceptor, a change in the overall color or other visualization of the test field may be apparent. For instance, a second acceptor could have a label of for instance a red fluorescence such as Texas Red®, and a first acceptor could have a label of for instance a green fluorescence such as fluoroscein, so that when first acceptor-blood substance complex and second acceptor are bound together in the test field, the color of fluorescence visualized therein would be yellow. Similarly, in another embodiment of the invention, both the first and the second acceptor may be labelled with for instance different colored latex particles. In such an embodiment, second acceptor may be labelled for instance with yellow latex particles, and first acceptor may be labelled for instance with blue latex particles. Prior to application of a feces sample, a yellow color would be visualizable in the test field (where second acceptor is immobilized). Binding of a blue-labelled first acceptor-substance complex to immobilized second acceptor would cause the yellow test field color to change to green (as blue + yellow = green).

In yet another embodiment of the invention, the visualization property of the first acceptor causes a detectable physical property different from background or other detectable property already existing in a test field by causing causing a decrease of color or other visualizable signal when detection occurs in a DTA, by quenching (diminishing) a visualization property of a second acceptor.

Also, as discussed above, a second acceptor specifically binds to or is bound by a portion of the substance to be detected by the DTA at a different location than a first acceptor. This is because both the first acceptor and the second acceptor bind or are bound to the substance, preferably blood substance, to be detected by a DTA simultaneously in the test field. As discussed above, such specific binding is typically not easily reversible, and is preferably considered irreversible in the usual meaning of the word in this context.

In yet another embodiment of this invention, the second acceptor immobilized at the test field may be bound to an additional acceptor or "competitive acceptor". Said competitive acceptor has a visualization property, preferably a label, and is bound to the second acceptor so that a DTA that has not been used will show a color or other visualizable signal in the test field. When a prepared feces sample is applied to a DTA of this invention, the additional acceptor is competitively removed and washed away if a complex formed by first acceptor and blood substance is present, by the specific binding of said complex to said second acceptor.

In such an embodiment, the first acceptor does not necessarily have a visualization property. Where the first acceptor does not have a visualization property, the visualizable signal in the test field will be diminished or even entirely removed upon application of a prepared feces sample having detectable amount of the substance to be detected. In a further such embodiment, the first acceptor may instead be conjugated to a "quenching" agent that will quench (diminish or destroy) the visualization property of the competitive acceptor.

In such an embodiment, when the first acceptor does have a visualization property, the visualizable signal in the test field will be altered. For instance, a competitive acceptor labelled with yellow latex particles would show a yellow color in the test field prior to application of sample. Said yellow color would fade or disappear (first acceptor without visualization property) or would change color to green (first acceptor labelled with blue latex particles) upon application of a prepared feces sample containing detectable amount of the substance to be detected.

The term "third acceptor" includes but is not limited to an antibody, antigen, hapten, immunoglobulin, T-cell, or any chemical or biological substance having a specific binding affinity for, or that will be specifically bound by a specific fecal matter component in the feces sample, including for instance bilirubin, sercobilin, stercobilinogen, normal fecal antigen (NFA), normal fecal antigen-1 (NFA-1), normal fecal antigen -2 (NFA-2), NCA-2 (non-specific cross-reacting antigen-2), *E*. *Coli*, fecal elastase, and other ubiquitous bacteria. If present, the third acceptor is immobilized in the first control field of a DTA, preferably in the shape of a line.

The term "fourth acceptor" includes but is not limited to an antibody, antigen, hapten, immunoglobulin, T-cell, or any chemical or biological substance having a specific binding affinity for, or that will be specifically bound by, first acceptor, second control acceptor, or a feces sample substance other than a specific component of fecal matter which has specific binding affinity for the third acceptor, or the blood substance to be detected by the detection test assembly, including for instance albumin, starch, or a component of a buffer solution used to prepare the feces sample. If present, the fourth acceptor is immobilized in the second control field of a DTA, preferably in the shape of a line.

Second, third, and fourth acceptors are preferably applied in a shape of a line. This results in colored lines at the test field (22), the first control field (44), and the second control field (24). Such acceptors may also be applied in other shapes, including shapes where the acceptors form circles, dots or figures.

The term "first control acceptor" includes but is not limited to an antibody, antigen, hapten, immunoglobulin, T-cell, or any chemical or biological substance having a specific binding affinity for a ubiquitous component of fecal matter, or that will be specifically bound by a ubiquitous component of fecal matter, creating a ubiquitous fecal matter component - first control acceptor complex. The ubiquitous fecal matter component in the feces sample, includes for instance bilirubin, sercobilin, stercobilinogen, normal fecal antigen (NFA), normal fecal antigen-1 (NFA-1), normal fecal antigen-2 (NFA-2), non-specific cross-reacting antigen-2 (NCA-2), *E*. *Coli*, fecal elastase, and other ubiquitous bacteria. If present, the first control acceptor is mobile and is localized in the reaction field of a DTA. When a feces sample is applied to a DTA and flows through the DTA in a downstream direction, the first control acceptor binds with the ubiquitous component of the fecal matter to create the ubiquitous fecal matter component - first control acceptor complex and moves with the feces sample downstream. The first control acceptor does not have a specific binding affinity for or by the substance to be detected by the DTA, the first acceptor, or the first acceptor-substance complex, the second acceptor, or the third acceptor. However, the first control acceptor has a specific binding affinity for the ubiquitous fecal matter component or will be specifically bound by said ubiquitous fecal matter component. The ubiquitous fecal matter component -first control acceptor complex bind to the third acceptor via the ubiquitous fecal matter component. Like the first acceptor, the first control acceptor is labelled or otherwise has a visualization property so that accumulation of the first control acceptor in the control field will result in the formation of color, preferably a colored line (control line) as otherwise discussed throughout this application.

The term "second control acceptor" includes but is not limited to an antibody, antigen, hapten, immunoglobulin, T-cell, or any chemical or biological substance having a specific binding affinity for, or that will be specifically bound by the fourth acceptor. If present, the second control acceptor is mobile and is localized in the reaction field of a DTA. When a feces sample is applied to a DTA and flows through the DTA in a downstream direction, the second control acceptor may move with the feces sample downstream. The second control acceptor does not have specific binding affinity for or by the substance to be detected by the DTA, the first acceptor, or the first acceptor-substance complex, the second acceptor, the specific fecal matter component, the ubiquitous fecal matter component - first control acceptor complex, or the third acceptor. However, the second control acceptor either has a specific binding affinity for fourth acceptor (immobilized in the second control field) or will be specifically bound by said fourth acceptor. Like first control acceptor, the second control acceptor is labelled or otherwise has a visualization property so that accumulation of the second control acceptor in the second control field will result in the formation of color, preferably a colored line (second control line) as otherwise discussed throughout this application.

The term "supporting backing" means, but is not limited to DTA hardware that provides or helps provide support and structure to a DTA. Any or all DTA fields may be attached to a supporting backing, for instance by using an adhesive such as glue. A supporting backing may be made of any solid material, including but not limited to plastic, glass, paper. Preferably, a supporting backing is made of one or more polyester sheets.

The term "flow" means, but is not limited to, movement of feces sample through a DTA, as discussed throughout this application.

The term "mobile" or "mobilized" means able to freely move with the flow of feces sample through a DTA. The term "immobile" or "immobilized" means fixed in place, not mobile, unable to freely move with the flow of feces sample through a DTA. For instance, in the context of a DTA of this invention, a first acceptor is mobile in the reaction field and may therefore travel with feces sample as the sample flows downstream to test field. However, the second acceptor immobilized in the test field is immobilized, or fixed, onto the test field.

A test and/or first and second control fields preferably show a color different from the color of the field in the presence of the substance to be detected by DTA ("positive result") and when the DTA is functioning correctly (first and second control lines) regardless of presence of substance. For instance, the different color forms as the first acceptor-substance complex or the first acceptor alone specifically bind to or is bound by immobilized second acceptor (test field) or immobilized third acceptor (first control field), respectively, and accumulates in a pattern defined by the pattern of the immobilized second or third acceptor in the test or first control field, respectively. Preferably, the pattern is in a straight line.

The sensitivity of a given DTA will vary depending on the substance, preferably the blood substance, to be detected in a feces sample by the DTA. For instance, where human hemoglobin (hHb) is the blood substance to be detected in a DTA, the sensitivity of the DTA from hHb may be as low as 0.05 ug hHb/ml feces sample. A sensitive DTA is not always preferred, for instance because normal baseline amounts of blood in a subject's fecal matter may then be detected, indicating a possible disease state to a user where none exists. Consider, for example, a DTA to test for hHb. As discussed above, nearly all patients with colorectal cancer exhibit fecal hHb concentrations of > 2 mg/g stool. A DTA for colorectal cancer patients may be made with a less sensitive detection of hHb therefore, to test for higher levels of hHb only.

The term "housing" means any solid body covering and protecting a DTA strip from external forces, including but not limited to for instance light, mechanical forces, physical contact such as touching, exposure to water, and to otherwise facilitate handling. A plastic cassette as shown in Figs. 3 and 4 is a preferred housing, with windows for easy application of feces sample and for easy reading of the results shown in the test field and control field. This cassette has a full and flat bottom side (not shown) in part to contain excess feces sample applied to the DTA. A housing may include or exclude windows, with DTA results read by an automatic system such as for instance an electronic reader that sends information relating to the result via an electronic signal for visual detection outside the housing (for instance, by lighting a "green" or a "red" light), or that sends such information to a computer or other electronic device that will display and/or record the result in another fashion. An alternative housing would hold a sample application field or reaction in the buffer solution or water in the vial containing feces sample at an upright angle. Once the feces sample was prepared, the feces sample could be applied to a DTA simply by having the DTA held in place preferably with the sample application field immersed in the feces sample and flow moving up the DTA toward a test field.

The present invention is also directed to a kit comprising a DTA of this invention as well as at least one of an instrument for transferring fecal matter, a container, a buffer solution, and a housing as discussed throughout this application. Preferably, the container has a means for measuring the buffer solution. More preferably, the buffer solution is pre-measured into the container. Even more preferably, the container may be closed so that buffer solution and fecal matter therein may be mixed together by shaking the container.

The present invention is also directed to a method for detecting a substance, preferably a blood substance, in fecal matter comprising the steps of preparing a feces sample and applying the feces sample to the DTA.

Numerous modifications and variations of the present invention are possible in view of the disclosure set forth in this application; therefore, the invention may be used otherwise than as particularly described. The Figures included herein and expressly discussed below are included as illustrative embodiments of the present invention, but are not meant to limit the invention.

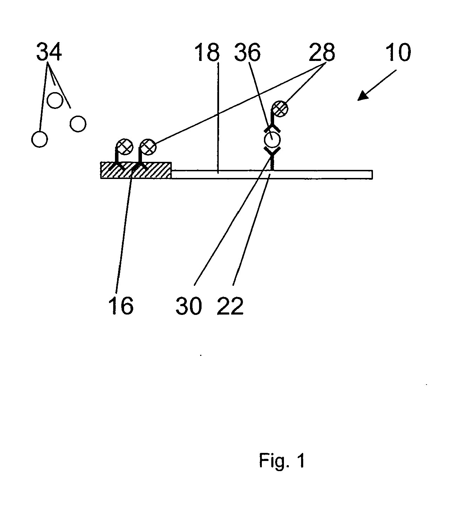

Fig. 1 shows a side view illustration of a DTA (10) of this invention. The DTA (10) has a reaction field (16) with mobile first acceptor (28), wherein the first acceptor (28) includes a chemical label having a visualization property (circular portion of first acceptor (28)). The DTA (10) also has a detection field (18) with a test field (22) wherein a second acceptor (30) is immobilized. A feces sample (34) having a blood substance (36) to be detected by the DTA (10) is applied directly to the reaction field (16). The feces sample (34) flows through the DTA (10) from the reaction field (16) to the detection field (18) and test field (22). During this flow, a blood substance (36) in the feces sample (34) encounters and specifically binds to the first acceptor (28). Upon reaching the test field (22), the first acceptor-blood substance complex binds via the blood substance (36) to the second acceptor (30). Accumulation of increasing amounts of first acceptor (28) in the test field (22) results in a change in test field color and a positive result (desired substance detected).

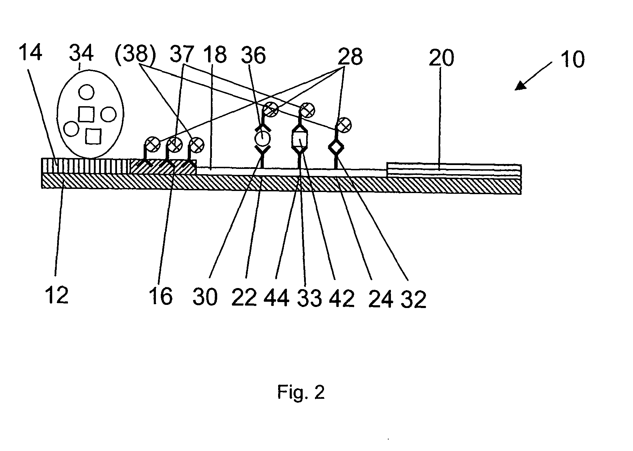

In Fig. 2, a preferred detection test assembly (10) for detecting a blood substance in a feces sample (34) comprises a supporting backing (12) made of for instance plastic on which is attached a sample application field (14), a reaction field (16), and a detection field (18). Said supporting backing (12) may be made of any solid material, including but not limited to plastic, glass or paper. The sample application field (14), the reaction field (16) and the detection field (18) may be made of material as discussed throughout this application.

After application of feces sample (34) to the sample application field (14), the sample (34) moves downstream to a reaction field (16) coated with a first acceptor (28) by capillary action. The first acceptor (28) has a specific binding affinity for the blood substance to be detected by the DTA and binds to the substance to form a first acceptor-substance complex. For instance, if the blood substance to be detected is hemoglobin, the first acceptor may be an anti-beta-hemoglobin antibody. "Downstream" is considered in the direction from the reaction field to the test field (22). In a positive feces sample (a feces sample having a substance detectable by a detection test assembly of this invention) the blood substance (36) binds to the first acceptor (28) and forms a first acceptor-hemoglobin complex. This complex moves downstream to a test field (22) within a detection field (18). The test field (22) has an immobilized second acceptor (30) specific for a blood substance (36), in this case hemoglobin. The second acceptor (30) may be a second anti-hemoglobin antibody having an affinity for the blood substance (36) (hemoglobin in this example) in different epitope than the first acceptor (28), for instance such as to the hemoglobin alpha chain. In a positive feces sample, as shown here, the first acceptor-hemoglobin complex binds to the second acceptor (30). Accumulation of increasing amounts of first acceptor (28) having a colored chemical label as a visualiation property in the test field (22) results in a change in test field color and a positive result.

Fig. 2 also illustrates a first control field (44) and a second control field (24), both as positive internal controls.

A mobile first control acceptor (37) having a visualization property at the reaction field (16), independent of the presence of a blood substance (36) in feces sample (34), moves with feces sample flow through the reaction field. During this flow, a ubiquitous sample component (42) in the feces sample (34) encounters and specifically binds to the mobile first control acceptor (37) creating a ubiquitous sample component -first control acceptor complex. The complex moves through the test field (22) to the first control field (44) which is within the detection field (18) and comprises an immobilized third acceptor (33) having a specific binding affinity for ubiquitous sample component (42) of the ubiquitous sample component - first control acceptor complex. Independent of the presence of a blood substance (36), the first control acceptor (37) and the third acceptor (33) bind in the presence of the ubiquitous sample component (42). Accumulation of increasing amounts of first control acceptor (37) in the first control field (44) results in a change in first control field (44) color and a first control line, where the visualization property is a color property such as colloidal gold.

First acceptor (28) that did not form a first acceptor-substance complex flows through the test field (22) to a second control field (24). The second control field (24) is within the detection field (18) and comprises an immobilized fourth acceptor (32) having a specific binding affinity for the first acceptor (28). Independent of the presence of a blood substance (36) the first acceptor (28) and the fourth acceptor (32) bind. Accumulation of increasing amounts of first acceptor (28) in the second control field (24) results in a change in second control field (24) color and a second control line.

As an alternative for an internal positive control, a mobile second control acceptor (38) having a visualization property at the reaction field (16), independent of the presence of a blood substance (36) in feces sample (34), moves with feces sample flow to the second control field (24). The second control field (24) is within the detection field (18) and comprises an immobilized fourth acceptor (32) having a specific binding affinity for the second control acceptor (38). Independent of the presence of a blood substance (36) the second control acceptor (38) and the fourth acceptor (32) bind. Accumulation of increasing amounts of second control acceptor (38) in the second control field (24) results in a change in second control field (24) color and a second control line, where the visualization property is a color property such as colloidal gold.

Fig. 2 also shows a DTA (10) having a soaking field (20) downstream of the detection field (18) to facilitate flow of the feces sample (34) through the DTA (10). Not being bound by theory, the dry soaking field helps draw the feces sample (34) to the soaking field after the feces sample (34) has flowed through and wetted the detection field (18). A tape may be applied at the border of the reaction field (16) and the detection field (18) to facilitate movement of the first acceptor (28) from the reaction field (16) by assisting feces sample (34) flow onto the detection field (18) by the tape (26).

Fig. 3 shows a working example and preferred embodiment of a DTA of the present invention. To make the DTA (10), a reaction field (16) made of a flat sheet of fiberglass membrane, was soaked with Murine Anti-beta-hHb Monoclonal Antibody-Colloidal Gold Conjugate (1 mg/ml) (see first acceptor (28) of Figs. 1 and 2), Rabbit IgG Antibody-Colloidal Gold Conjugate (1 mg/ml) (see second control acceptor (38) of Fig. 2), and Carcinoembryonic Antigen (CEA) Colloidal Gold Conjugate (1mg/ml) (see first control acceptor (37) of Fig. 2). While both first acceptor and second acceptor in this example were labelled with colloidal gold, the invention also includes a DTA (10) where two different labels are used. Goat Anti-alpha-hHb Monoclonal Antibody (1 mg/ml) (see second acceptor (30) of Figs. 1 and 2), Goat Anti-Rabbit IgG Polyclonal Antibody (1 mg/ml) (see fourth acceptor (32) of Fig.2), and Carcinoembryonic Antigen (CEA) Antibody (1mg/ml) (see third acceptor (33) of Fig. 2) were coated in the shape of lines onto a sheet of nitrocellulose membrane (see detection field (18) of Fig. 2)). Both the reaction and detection fields were subsequently conventionally dried, but freeze drying can be used as an alternative. A sample application field (14), also made of a fibreglass membrane, and the reaction field (16) were glued to a supporting backing (12) made of polyester sheets. The detection field (18) and a soaking field (20) made of a porous paper were also glued to the supporting backing. The supporting backing was obtained from G & L (San Jose, California); membranes were obtained from Whatman (Kent, UK) and S&S (Keene, New Hampshire); antibodies were obtained from Lumiquick Diagnostics (Santa Clara, California). A Scotch tape adhesive (26) was applied to the DTA, covering the borderline between the reaction field and the detection field. The sheet comprising the supporting backing and glued-on fields was cut into 3.8 mm wide strips. The sensitivity of the DTA resulted in a weak test line at a concentration of 0.05 µg hHb/ml. A buffer solution containing Bovine Serum Albumin, Sodium Azide, Sodium Dibasic Phosphate and Sodium Chloride (all Sigma Chemicals, St. Louis, MO) was prepared and spiked to 0.05 µg/ml with hHB (Sigma). (Buffer content: 1 L H2O, 1g BSA, 1.022 g Na2HP04, 0.336g NaH2PO4, 8.766g NaCl, 1 g NaN3, 0.05ml Tween 20, pH 7.4). The Tween 20 in particular need not be included in the buffer solution.

A feces sample was applied to the sample application field (14). To prepare the feces sample, fresh fecal matter was taken from three different sites of a fresh human stool by picking at the sites with a long toothpick-like instrument having a small flat edge. The fecal matter at each taking weighed, on average, 11.5mg. With each of the three takings, the instrument was dipped into a vial containing 2.5 ml of the buffer solution described above. All of these takings were dipped into the same vial. In total, 34.5 mg fecal matter was added to the vial. The vial was then capped and shaken to mix the fecal matter with the buffer solution. Most of the fecal matter dissolved in the buffer.

Two drops of the feces sample were applied to the DTA sample application field (14). A positive result in the test field (22) and a control line in the first control field (44) and the second control field (24) were both visible to the naked eye approximately five minutes after application of the feces sample, and the photograph of Fig. 3 was taken soon thereafter.

In the context of the present invention, soaking (for instance fiberglass) is different from coating (for instance nitrocellulose). Soaking occurs when a membrane is dipped into a solution or otherwise has solution applied in a gross manner, without regard to a specific shape such as a line. Coating occurs when a solution is applied for instance by spraying or with a brush, in part to control the shape in which the coat is applied. Most preferably, non-labelled antibodies are coated, not soaked, as they are applied in a desired shape such as a line.

As various changes could be made in the above-described DTA, kits and methods without departing from the scope of the invention, it is intended that all matter contained in the above description be interpreted as illustrative and not in a limiting sense.

## Claims

1. A detection test assembly for detecting a substance in a sample, the substance having a first substance portion and a second substance portion, comprising:
a reaction field comprising a first acceptor, wherein said first acceptor is mobile and has a visualization property, and wherein a specific binding affinity exists between said first acceptor and the first portion of said substance such that a first acceptor- substance complex may be formed; and
a detection field comprising a test field, and at least a first control field, wherein the test field has a second acceptor, wherein said second acceptor is immobilized on the test field and wherein a specific binding affinity exists between at least one of:
(i) said second acceptor; and at least one of (a) the second portion of said substance and (b) a portion of the first acceptor- substance complex formed by first acceptor and the first portion of said substance; and
wherein the first control field is connected to the test field, wherein the first control field has an immobilized third acceptor, and wherein said third acceptor has a specific binding affinity for a ubiquitous sample component, other than the substance to be detected by the detection test assembly.

2. The detection test assembly of claim 1, further comprising a second control field connected to the first control field or the test field, wherein the second control field has an immobilized fourth acceptor, and wherein said fourth acceptor has a specific binding affinity for a component in the detection test assembly, other than the substance to be detected by the detection test assembly or the ubiquitous sample component to be detected by the third acceptor, and having a visualization property.

3. The detection assembly of claim 1, wherein said first portion of said substance is on a location different from said second portion of said substance so that said first acceptor, said second acceptor and said substance may be specifically bound together simultaneously.

4. The detection assembly of claim 1, wherein said first acceptor does not have a specific binding affinity for said second acceptor and said second acceptor does not have a specific binding affinity for said first acceptor; and wherein the test field is connected to and located downstream from the reaction field.

5. The detection test assembly of claim 1, further comprising a supporting backing.

6. The detection test assembly of claim 1, further comprising a sample application field connected to and located upstream of the reaction field.

7. The detection test assembly of claim 1 further comprising a soaking field connected to and located downstream from the detection field, wherein said soaking field comprises a porous material for absorbing an excess of the sample.

8. The detection test assembly of claim 1, wherein the visualization property of said first acceptor is a label conjugated to the first acceptor.

9. The detection test assembly of claim 8, wherein said label is selected from the group consisting of colloidal gold, colored latex particle, magnetic particle, and a chemical color dye.

10. The detection test assembly of claim 1, wherein said first acceptor and said second acceptor are, individually, selected from the group consisting of monoclonal antibody, polyclonal antibody, and a combination of monoclonal antibody and polyclonal antibody.

11. The detection test assembly of claim 10 wherein said first acceptor is anti-beta-human hemoglobin monoclonal antibody labelled with colloidal gold and binds to said second acceptor which is an anti-alpha-human hemoglobin monoclonal antibody.

12. The detection test assembly of claim 11 further comprising a second control field connected to and located downstream from the test field and having an immobilized fourth acceptor that is an antibody having a specific binding affinity for the first acceptor.

13. The detection test assembly of claim 12 further comprising a sample application field and a soaking field, wherein said sample application field and said reaction field seamlessly and directly abut and are made of fibreglass, wherein said detection field seamlessly and directly abuts said reaction field and is made of nitrocellulose membrane, and said soaking field seamlessly and directly abuts said detection field and is made of one of the group selected from porous paper and silica gel matrix.

14. A method of using the detection test assembly of claim 1, comprising at least the steps of:
preparing a sample by transferring the substance with an instrument into a container,
providing a buffer solution in the said container, wherein said buffer solution has a pH of about 4 to 10, more preferably of about 6 to 8, more preferably of about 7 to 7.8, and most preferably of about 7.4, and contains a preservative; and
applying the sample to a sample application field.

15. The detection test assembly of claim 1, wherein the ubiquitous sample component binds to a mobile first control acceptor, having a visualization property, located in the reaction field, creating a ubiquitous sample component - first control acceptor complex.
